# EUROPÄISCHE PATENTANMELDUNG

(11) **EP 4 239 048 A1**
(43) Veröffentlichungstag der Anmeldung: **06.09.2023**
(21) Anmeldenummer: 22159932.7
(22) Anmeldetag: 03.03.2022
(51) Int. Cl.: C11D 3/22, C11D 17/00, A61L 9/05

(54) **EINSATZ VON CYCLODEXTRINEN IN WC-PRODUKTEN ZUR BESEITIGUNG VON SCHLECHTGERÜCHEN**

(71) Anmelder: Henkel AG & Co. KGaA, 40589 Düsseldorf (DE)
(72) Erfinder: WEYHE, Marc, 47802 Krefeld (DE); SCHEUER, Madeline, 47475 Kamp-Lintfort (DE); PESSEL, Frank, 40215 Düsseldorf (DE)

(57) **Zusammenfassung**

Die Erfindung betrifft ein WC-Produkt, umfassend mindestens ein Cyclodextrin. Ferner betrifft die Erfindung die Verwendung des WC-Produktes zur Reduzierung von Schlechtgerüchen und/oder zur Beduftung, ein Verfahren zur Reduzierung von Schlechtgerüchen und/oder zur Beduftung, wobei in mindestens einem Verfahrensschritt ein erfindungsgemäßes WC-Produkt angewendet wird, und die Verwendung von mindestens einem Cyclodextrin zur Reduzierung von Schlechtgerüchen und/oder zur Verbesserung des Dufteindrucks eines Produktes, in dem das Cyclodextrin enthalten ist, wobei das Cyclodextrin in einem WC-Produkt enthalten ist.

## Beschreibung

Die Erfindung betrifft ein WC-Produkt, umfassend mindestens ein Cyclodextrin. Ferner betrifft die Erfindung die Verwendung des WC-Produktes zur Reduzierung von Schlechtgerüchen und/oder zur Beduftung, ein Verfahren zur Reduzierung von Schlechtgerüchen und/oder zur Beduftung, wobei in mindestens einem Verfahrensschritt ein erfindungsgemäßes WC-Produkt angewendet wird, und die Verwendung von mindestens einem Cyclodextrin zur Reduzierung von Schlechtgerüchen und/oder zur Verbesserung des Dufteindrucks eines Produktes, in dem das Cyclodextrin enthalten ist, wobei das Cyclodextrin in einem WC-Produkt enthalten ist.

Im Bereich Home Care gibt es viele intensive und unangenehm riechende Verbindungen wie z.B. Toilettenschlechtgerüche, die beim Benutzen der Toilette auftreten. Zudem wurde festgestellt, dass sich diese Verbindungen und der daraus resultierende Schlechtgeruch bei Nicht-Benutzung der Toilette über einen längeren Zeitraum, beispielsweise mehrere Tage, Wochen oder sogar länger, halten können.

Typischerweise werden in Reinigern und Lufterfrischern zur Überdeckung der Schlechtgerüche Parfüms angewendet. Deren Wirkung reicht aber in vielen Fällen nicht aus, um die Schlechtgerüche vollständig zu überdecken oder aber die Zugabe einer ausreichenden Menge an Parfümstoffen stellt einen großen Kostenpunkt bei der Herstellung der Produkte dar. Auch sind einige Riechstoffe nicht immer erhältlich oder die einzusetzende Menge der Parfümstoffe wird durch die Formulierbarkeit in die Produktmatrix limitiert. Deshalb ist es wünschenswert, negative Dufteindrücke auf anderem Wege als durch die Überdosierung von Parfümstoffen zu lösen.

Überraschenderweise wurde gefunden, dass Cyclodextrine, die beispielsweise bestehenden Parfümierungen oder Produkt-Matrizes zugegeben werden, eine starke Wirkung gegen Schlechtgerüche aufweisen und so zu einem verbesserten olfaktorischen Eindruck bei der Anwendung des Produktes führen. Insbesondere führt der Einsatz von Cyclodextrinen als Wirkstoff zur Verhinderung/Beseitigung von Schlechtgerüchen (Malodor Counteracting Agent) in oder auf einem WC-Produkt, bevorzugt einem WC-Reiniger oder WC-Erfrischer wie einem WC-Stein (SVR), zu einer besseren Schlechtgeruchsreduzierung im Vergleich zu Produkten ohne Cyclodextrine. Bevorzugt führt der Einsatz von Cyclodextrinen auch zu einem verbesserten Dufteindruck des Produktes, insbesondere einer besseren Parfümintensität.

Somit betrifft die Erfindung in einem ersten Aspekt ein WC-Produkt, umfassend mindestens ein Cyclodextrin.

In bevorzugten Ausführungsformen ist das WC-Produkt zur Reduzierung von Schlechtgerüchen und/oder zur Beduftung geeignet.

Besonders bevorzugt ist das WC-Produkt ein WC-Stein.

In bevorzugten Ausführungsformen ist das mindestens eine Cyclodextrin ein β-Cyclodextrin, ein alkyliertes β-Cyclodextrin oder ein hydroxyalkyliertes β-Cyclodextrin, beispielsweise Methyl-β-Cyclodextrin oder Hydroxypropyl-β-Cyclodextrin.

In einem weiteren Aspekt betrifft die Erfindung die Verwendung des erfindungsgemäßen WC-Produktes zur Reduzierung von Schlechtgerüchen und/oder zur Beduftung.

Ein weiterer Aspekt der Erfindung betrifft ein Verfahren zur Reduzierung von Schlechtgerüchen und/oder zur Beduftung, wobei in mindestens einem Verfahrensschritt ein erfindungsgemäßes WC-Produkt angewendet wird.

Schließlich betrifft die Erfindung in einem letzten Aspekt die Verwendung von mindestens einem Cyclodextrin zur Reduzierung von Schlechtgerüchen und/oder zur Verbesserung des Dufteindrucks eines WC-Produktes, in dem das Cyclodextrin enthalten ist.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

"Mindestens ein", wie hierin verwendet, schließt ein, ist aber nicht begrenzt auf 1, 2, 3, 4, 5, 6 und mehr. Bezogen auf einen Inhaltsstoff bezieht sich die Angabe auf die Art des Inhaltsstoffs und nicht auf die absolute Zahl der Moleküle. "Mindestens ein Cyclodextrin" bedeutet somit beispielsweise mindestens eine Art von Cyclodextrin, d.h. dass eine Art von Cyclodextrin oder eine Mischung mehrerer verschiedener Cyclodextrine gemeint sein kann. Zusammen mit Gewichtsangaben bezieht sich die Angabe auf alle Verbindungen der angegebenen Art, die in dem Produkt enthalten sind, d.h. dass das Produkt über die angegebene Menge der entsprechenden Verbindungen hinaus keine weiteren Verbindungen dieser Art enthält.

"Ungefähr" oder "etwa", wie hierin in Bezug auf Zahlenwerte verwendet, bedeutet den entsprechenden Wert ±10%, vorzugsweise ±5%, stärker bevorzugt ±3%, am stärksten bevorzugt ±1%.

Gemäß der Erfindung zeichnet sich das im Folgenden detaillierter beschriebene WC-Produkt dadurch aus, dass es mindestens ein Cyclodextrin umfasst und bevorzugt zur Reduzierung von Schlechtgerüchen und/oder zur Beduftung geeignet ist.

In verschiedenen Ausführungsformen ist es möglich, dass die Schlechtgerüche nicht nur reduziert, sondern (nahezu) vollständig beseitigt werden. Somit betrifft die Erfindung in einem weiteren Aspekt auch ein WC-Produkt das bevorzugt zur Beseitigung von Schlechtgerüchen und/oder zur Beduftung geeignet ist, wobei das WC-Produkt mindestens ein Cyclodextrin umfasst.

Die Begriffe "WC-Produkt", "WC-Artikel" oder "Toilettenzusatzartikel" sind im Kontext dieser Erfindung synonym zu verwenden.

Bevorzugt ist das WC-Produkt ein WC-Reiniger oder ein WC-Erfrischer, z.B. ein WC-Lufterfrischer, stärker bevorzugt ein WC-Stein oder ein Duftspüler, insbesondere ein WC-Stein (SVR).

Die Begriffe "WC-Stein" oder "Klostein" sind im Kontext dieser Erfindung synonym zu verwenden und bezeichnen feste Zusammensetzungen, die in einer Toilette platziert/befestigt werden und die enthaltenen Inhaltstoffe über mehrere Spülvorgänge nach und nach in das Spülwasser freisetzen.. Die Abkürzung SVR steht dabei für superior value rim block.

In verschiedenen Ausführungsformen ist das WC-Produkt vorzugsweise ein Produkt, das in der Toilette angewendet wird, insbesondere in dieser zur Dauerbenutzung angebracht wird, wie beispielsweise der oben beschriebene WC-Stein oder ein Duftspüler. Es kann aber auch ein WC-Reiniger sein, der beispielsweise zur Reinigung und/oder Desinfektion und/oder Beduftung der Toilette angewendet wird und nicht dauerhaft eingesetzt wird. Es ist aber auch denkbar, dass das WC-Produkt ein Lufterfrischer ist, der außerhalb der Toilette aufgestellt wird und bevorzugt Schlechtgerüche im Raum reduziert oder beseitigt, und/oder den Raum beduftet.

Das WC-Produkt kann dabei fest oder flüssig sein.

"Flüssig", wie hierin verwendet, schließt Flüssigkeiten und Gele sowie auch pastöse Zusammensetzungen ein. Diese Angabe bezieht sich, sofern nicht anders angegeben, auf Standardbedingungen, d.h. einen Druck von 1 bar und eine Temperatur von 20°C. Bevorzugt sind die flüssigen Zusammensetzungen bei Raumtemperatur (20 °C) fließfähig und gießbar, es ist aber auch möglich, dass sie eine Fließgrenze aufweisen. In verschiedenen Ausführungsformen kann das WC-Produkt ein Gel sein, beispielsweise ein Gel mit einer Fließgrenze.

"Fest", wie hierin verwendet, bezeichnet Zusammensetzungen, die bei Raumtemperatur (20 °C) und bei Standarddruck (1 bar) fest sind, also nicht flüssig oder gasförmig.

In verschiedenen Ausführungsformen können die erfindungsgemäßen WC-Produkte beispielsweise, ohne darauf beschränkt zu sein, in (Kunststoff-)Behältnisse, insbesondere Mehrkammerbehältnisse, gefüllt werden, um dauerhaft in der Toilette angebracht zu werden. Auch das Befüllen eines Applikators, um das Produkt innen auf die Toilettenschüssel aufzustempeln ist denkbar.

In bevorzugten Ausführungsformen ist das WC-Produkt fest, insbesondere ist es ein fester WC-Stein.

In bevorzugten Ausführungsformen wird das Cyclodextrin des erfindungsgemäßen WC-Produktes bei Kontakt mit Wasser, beispielsweise beim Abspülen in der Toilette, freigesetzt. Dabei kann es sich in der Toilette und optional auch im Raum verteilen und vorzugsweise mit Schlechtgeruch-verursachenden, typischerweise meist flüchtige Verbindungen (Malodors) wechselwirken, z.B. diese binden, adsorbieren, komplexieren, oxidieren oder mit ihnen Einschlussverbindungen bilden, so dass sie geruchlich inaktiviert werden. Dabei sollen die Schlechtgeruch-verursachenden Verbindungen bevorzugt möglichst lange an das Cyclodextrin gebunden bleiben und nicht wieder freigesetzt werden, wodurch sich der Schlechtgeruch in der Toilette und im umgebenden Raum reduziert oder sogar vollständig beseitigt wird. Neben der Reduzierung oder Beseitigung von Schlechtgerüchen kann das erfindungsgemäße Produkt auch einen verbesserten Dufteindruck hervorrufen, insbesondere eine verbesserte Parfümintensität, verglichen mit WC-Produkten, die kein Cyclodextrin enthalten.

In verschiedenen Ausführungsformen kann sich die Verbesserung des Dufteindruckes dabei auf das Produkt selbst beziehen, beispielsweise der Duft des Produktes vor der Anwendung oder wenn es über einige Zeit nicht angewendet wurde. Bevorzugt ist aber der Dufteindruck gemeint, der durch die Anwendung des Produktes, beispielsweise beim Abspülen der Toilette, hervorgerufen wird und der bevorzugt über einige Zeit in der Toilette und optional auch im umgebenden Raum aufrechterhalten wird.

Es ist bevorzugt, dass die Anwendung des erfindungsgemäßen WC-Produktes, insbesondere des (festen) WC-Steins, einen vorliegenden Schlechtgeruch um mindestens 5 % reduziert, stärker bevorzugt um mindestens 10 %, noch stärker bevorzugt um mindestens 15 %, noch stärker bevorzugt um mindestens 20 %, noch stärker bevorzugt um mindestens 25 %, noch stärker bevorzugt um mindestens 30 %, noch stärker bevorzugt um mindestens 35 %, noch stärker bevorzugt um mindestens 40 %, noch stärker bevorzugt um mindestens 45 %, noch stärker bevorzugt um mindestens 50 %, noch stärker bevorzugt um mindestens 55 %, noch stärker bevorzugt um mindestens 60 %, noch stärker bevorzugt um mindestens 65 %, noch stärker bevorzugt um mindestens 70 %, noch stärker bevorzugt um mindestens 75 %, noch stärker bevorzugt um mindestens 80 %, noch stärker bevorzugt um mindestens 85 %, noch stärker bevorzugt um mindestens 90 %, noch stärker bevorzugt um mindestens 91 %, noch stärker bevorzugt um mindestens 92 %, noch stärker bevorzugt um mindestens 93 %, noch stärker bevorzugt um mindestens 94 %, noch stärker bevorzugt um mindestens 95 %, noch stärker bevorzugt um mindestens 96 %, noch stärker bevorzugt um mindestens 97 %, noch stärker bevorzugt um mindestens 98 %, noch stärker bevorzugt um mindestens 99 % und insbesondere um 100 %, wobei mit 100 % die vollständige Beseitigung des Schlechtgeruchs gemeint ist.

In besonders bevorzugte Ausführungsformen wird der Schlechtgeruch um mindestens 25 % reduziert, bevorzugt um mindestens 26 %, stärker bevorzugt um mindestens 30 %, stärker bevorzugt um mindestens 33 %, noch stärker bevorzugt um mindestens 40 %, noch stärker bevorzugt um mindestens 42 %, noch stärker bevorzugt um mindestens 50 %, noch stärker bevorzugt um mindestens 52 %, noch stärker bevorzugt um mindestens 55 %, noch stärker bevorzugt um mindestens 56 %, noch stärker bevorzugt um mindestens 60 % und insbesondere um mindestens 63 %.

In verschiedenen Ausführungsformen geht die Reduzierung des Schlechtgeruches beispielsweise so weit, dass der Schlechtgeruch (nahezu) vollständig beseitigt wird, bevorzugt bedeutet das, dass der Schlechtgeruch zu mindestens 95 % beseitigt wird, stärker bevorzugt zu mindestens 96 %, noch stärker bevorzugt zu mindestens 97 %, noch stärker bevorzugt zu mindestens 98 %, noch stärker bevorzugt zu mindestens 99% und insbesondere zu 100 %.

Die Reduzierung des Schlechtgeruchs oder die Beseitigung des Schlechtgeruchs und/oder die Beduftung (beispielsweise der verbesserte Dufteindruck des Produkts), insbesondere die Wirkung der Cyclodextrine, hält bevorzugt mindestens 5 Minuten an, stärker bevorzugt mindestens 10 Minuten, noch stärker bevorzugt mindestens 30 Minuten, noch stärker bevorzugt mindestens eine Stunde (60 Minuten), noch stärker bevorzugt mindestens drei Stunden, noch stärker bevorzugt mindestens fünf Stunden, noch stärker bevorzugt mindestens 10 Stunden, noch stärker bevorzugt mindestens 15 Stunden, noch stärker bevorzugt mindestens 24 Stunden, noch stärker bevorzugt mindestens 2 Tage (48 Stunden), noch stärker bevorzugt mindestens 3 Tage, noch stärker bevorzugt mindestens 4 Tage, noch stärker bevorzugt mindestens 5 Tage, noch stärker bevorzugt mindestens 6 Tage und insbesondere mindestens 7 Tage oder länger, beispielsweise mindestens 8 Tage, mindestens 9 Tage, mindestens 10 Tage, mindestens 11 Tage, mindestens 12 Tage, mindestens 13 Tage oder mindestens 14 Tage oder länger. Dabei beginnt der angegebene Zeitraum typischerweise, wenn nicht anders angegeben, direkt nach der Anwendung des Produktes, z.B. nach dem Abspülen bei einem in der Toilette angebrachten Produkt, oder z.B. nachdem ein WC-Reiniger in die Toilette gegeben wurde.

In bevorzugten Ausführungsformen ist das mindestens eine Cyclodextrin in dem WC-Produkt in einer Menge von 0,01 bis 30 Gew.-% enthalten, bevorzugt 0,01 bis 25 Gew.-%, stärker bevorzugt 0,01 bis 20 Gew.-%, noch stärker bevorzugt 0,01 bis 15 Gew.-%, noch stärker bevorzugt 0,01 bis 10 Gew.-%, insbesondere 0,01 bis 5 Gew.-%, beispielsweise 0,01 bis 2 Gew.-% oder 0,01 bis 1 Gew.-% wie z.B. 0,01 Gew.-%, 0,02 Gew.-%, 0,03 Gew.-% 0,04 Gew.-% 0,05 Gew.-%, 0,06 Gew.-%, 0,07 Gew.-%, 0,08 Gew.-% 0,09 Gew.-%, 0,1 Gew.-%, 0,2 Gew.-%, 0,3 Gew.-%, 0,4 Gew.-%, 0,5 Gew.-%, 0,6 Gew.-%, 0,7 Gew.-%, 0,8 Gew.-%, 0,9 Gew.-% oder 1 Gew.-%, bezogen auf das Gesamtgewicht (der Zusammensetzung) des WC-Produktes.

Das mindestens eine Cyclodextrin kann jede Art von bekannten Cyclodextrinen sein, beispielsweise substituierte und unsubstituierte Cyclodextrine, die etwa 6 bis etwa 12 Glukoseeinheiten, z.B. 6, 7 oder 8 Glukoseeinheiten, umfassen sowie Mischungen davon.

In verschiedenen Ausführungsformen ist das mindestens eine Cyclodextrin ein α-Cydodextrin, ein β-Cyclodextrin, ein γ-Cyclodextrin, ein alkyliertes α-Cydodextrin, ein alkyliertes β-Cyclodextrin, ein alkyliertes γ-Cyclodextrin, ein hydroxyalkyliertes α-Cydodextrin, ein hydroxyalkyliertes β-Cyclodextrin oder ein hydroxyalkyliertes γ-Cyclodextrin, oder eine Mischung davon ist.

Bevorzugt ist das mindestens eine Cyclodextrin β-Cyclodextrin, alkyliertes β-Cyclodextrin, hydroxyalkyliertes β-Cyclodextrin oder eine Mischung davon, insbesondere methyliertes β-Cyclodextrin oder hydroxyalkyliertes β-Cyclodextrin oder eine Mischung davon, beispielsweise Methyl-β-Cyclodextrin oder Hydroxypropyl-β-Cyclodextrin oder eine Mischung davon.

In besonders bevorzugten Ausführungsformen sind vor allem WC-Produkte wie feste WC-Steine, die methyliertes β-Cyclodextrin oder hydroxyalkyliertes β-Cyclodextrin, beispielsweise Methyl-β-Cyclodextrin oder Hydroxypropyl-β-Cyclodextrin, umfassen, zur Reduzierung von Schlechtgerüchen geeignet. Zusätzlich hinterlassen diese Produkte bei der Anwendung, insbesondere in der Toilette und optional im umgebenden Raum, einen verbesserten Dufteindruck, beispielsweise eine verbesserte Parfümintensität. Bevorzugt ist das mindestens eine Cyclodextrin in diesen Ausführungsformen in einer Menge von 0,01 bis 30 Gew.-%, stärker bevorzugt von 0,01 bis 20 Gew.-%, noch stärker bevorzugt von 0,01 bis 5 Gew.-%, und insbesondere in einer Menge von 0,05 Gew.-%, bezogen auf das Gesamtgewicht des WC-Produktes, in diesem enthalten.

Hydroxypropyl-β-Cyclodextrin kann beispielsweise unter dem Namen CAVASOL^{®} W7 HP PHARMA und Methyl-beta-Cyclodextrin unter dem Namen CAVASOL^{®} W7 M von der Firma Wacker Chemie AG bezogen werden.

Beispiele für Schlechtgeruch-verursachende Verbindungen sind, ohne darauf beschränkt zu sein, Amine wie Diethylamin, Triethylamin, Indol und Skatol, Sulfide wie Dimethylsulfid und Dimethyltrisulfid, Thiole wie Methylmercaptan und organische Säuren wie Buttersäure, Isovaleriansäure, Valeriansäure, Capronsäure, ungesättigte kurzkettige Carbonsäuren und ungesättigte oder hydroxylierte verzweigte Fettsäuren und Sulfanylalkanole. Eine bevorzugte Schlechtgeruchmischung im Kontext dieser Erfindung umfasst Sulfide wie Dimethylsulfid, Amine wie Indol und Skatol, und Säuren wie Buttersäure, Isovaleriansäure und Valeriansäure. In bevorzugten Ausführungsformen ist das erfindungsgemäße WC-Produkt besonders gegen einen Schlechtgeruch wirksam, der eine oder mehrere der genannten Verbindungen umfasst.

In verschiedenen Ausführungsformen enthält das WC-Produkt mindestens einen weiteren Bestandteil, bevorzugt mindestens zwei weitere Bestandteile, beispielsweise 2, 3, 4, 5 ,6 ,7, 8, 9 oder mehr weitere Bestandteile, ausgewählt aus der Gruppe bestehend aus Tensiden, Farbstoffen, Parfümstoffen, Abspülregulatoren, Bleichmitteln, Buildern, Säuren, Basen, Lösungsmitteln, antimikrobiellen Wirkstoffen, Polymeren, Salzen, Verdickungsmitteln, Konservierungsstoffen, Komplexbildnern, weiteren Wirkstoffen zur Verringerung von Schlechtgerüchen, die keine Cyclodextrine sind, Parfümboostern, Füllstoffen, Bleichmitteln, Korrosionsinhibitoren, Enzymen, Mikroorganismen, Wirkstoffen zur Biofilmentfernung, Wirkstoffen zur Inhibierung der Kalkablagerung, Wirkstoffen zur Verminderung der Schmutzhaftung, Wirkstoffen zur Verbesserung der Verarbeitbarkeit, Wirkstoffen zur Verringerung der Klebrigkeit und Mischungen davon.

Es ist besonders bevorzugt, dass das WC-Produkt mindestens einen Parfümstoff oder eine Mischung mehrerer Parfümstoffe umfasst.

Diese sind in dem Produkt vorzugsweise in einer Menge von 0,01 bis 10 Gew.-%, insbesondere 0,05 bis 8 Gew.-%, besonders bevorzugt 0,1 bis 5 Gew.-% enthalten. Als eine Parfümkomponente kann dabei d-Limonen enthalten sein. In einer besonders bevorzugten Ausführungsform enthält das Produkt dabei haftfeste Parfümstoffe, insbesondere ätherische Öle (auch als essentielle Öle bezeichnet). Als solche sind beispielsweise Pine-, Citrus-, Jasmin-, Patchouly-, Rosen- oder Ylang-Ylang-Öl im Sinne dieser Erfindung einsetzbar. Ebenfalls geeignet sind Muskateller-Salbeiöl, Kamillenöl, Lavendelöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Aber auch weitere haftfeste Parfümstoffe, etwa die höhersiedenden bzw. festen Parfümstoffe natürlichen oder synthetischen Ursprungs, oder auch leichter flüchtige Parfümstoffe, insbesondere die niedriger siedenden Parfümstoffe natürlichen oder synthetischen Ursprungs, die allein oder in Mischungen eingesetzt werden können, sind im Rahmen der vorliegenden Erfindung vorteilhaft einsetzbar.

In bevorzugten Ausführungsformen kann/können der eine eingesetzte Parfümstoff oder die eingesetzten Parfümstoffe mit dem mindestens einen Cyclodextrin synergistisch wirken und eine verbesserte Schlechtgeruch-Reduzierung und/oder einen verbesserten Dufteindruck des Produktes hervorrufen, verglichen mit Produkten, die nur einen der Bestandteile enthalten. In verschiedenen Ausführungsformen umfasst dieser Parfümstoff, welcher mit dem mindestens einen Cyclodextrin synergistisch zusammenwirkt, Limonen, z.B. d-Limonen.

Beispielsweise kann die Intensität des Schlechtgeruchs kurzfristig um mindestens 5 %, bevorzugt um mindestens 10 %, stärker bevorzugt um mindestens 15 %, noch stärker bevorzugt mindestens 20 %, noch stärker bevorzugt mindestens 25 %, noch stärker bevorzugt um mindestens 26 %, noch stärker bevorzugt um mindestens 30 % und insbesondere mindestens 33 %, beispielsweise mindestens 35 %, mindestens 40 %, mindestens 50 % oder mindestens 55 %, reduziert werden, verglichen mit Produkten die nur mindestens einen Parfümstoff aber kein Cyclodextrin enthalten.

In einer anderen Ausführungsform kann der kurzfristige Dufteindruck, insbesondere die Parfümintensität, des erfindungsgemäßen WC-Produktes um mindestens 4 %, bevorzugt um mindestens 4,5 %, stärker bevorzugt um mindestens 5 %, noch stärker bevorzugt um mindestens 6 %, noch stärker bevorzugt um mindestens 7 %, noch stärker bevorzugt um mindestens 8 %, noch stärker bevorzugt um mindestens 9 %, beispielsweise um mindestens 9,2 %, mindestens 10 %, mindestens 12 % oder mindestens 15 %, verbessert werden, verglichen mit Produkten, die nur mindestens einen Parfümstoff aber kein Cyclodextrin enthalten.

"Kurzfristig" bezieht sich hierbei bevorzugt auf eine Zeit nach der Anwendung des erfindungsgemäßen Produktes von mindestens 10 Sekunden, stärker bevorzugt von mindestens 30 Sekunden, noch stärker bevorzugt von mindestens einer Minute, noch stärker bevorzugt von mindestens zwei Minuten, noch stärker bevorzugt von mindestens 3 Minuten, noch stärker bevorzugt von mindestens 4 Minuten, noch stärker bevorzugt von mindestens 5 Minuten, beispielsweise mindestens 10 Minuten, mindestens 20 Minuten oder mindestens 1 Stunde und optional bis zu 5 Minuten, bis zu 30 Minuten, bis zu 1 Stunde, bis zu 2 Stunden, bis zu 5 Stunden, bis zu 10 Stunden oder bis zu einem Tag.

Langfristig, kann die Intensität des Schlechtgeruchs bevorzugt um mindestens 20 %, stärker bevorzugt um mindestens 25 %, noch stärker bevorzugt um mindestens 30 %, noch stärker bevorzugt um mindestens 35 %, noch stärker bevorzugt um mindestens 40 %, noch stärker bevorzugt um mindestens 42 %, noch stärker bevorzugt um mindestens 50 % und insbesondere um mindestens 52 %, beispielsweise um mindestens 55 %, mindestens 58 %, mindestens 60 % oder mindestens 65 %, reduziert werden, verglichen mit Produkten die nur mindestens einen Parfümstoff aber kein Cyclodextrin enthalten.

In anderen bevorzugten Ausführungsformen kann der langfristige Dufteindruck, insbesondere die Parfümintensität, des erfindungsgemäßen WC-Produktes um mindestens 50 %, bevorzugt um mindestens 60 %, stärker bevorzugt um mindestens 70 % noch stärker bevorzugt um mindestens 75 %, noch stärker bevorzugt um mindestens 80 %, noch stärker bevorzugt um mindestens 90 %, noch stärker bevorzugt um mindestens 100 % und insbesondere um mindestens 105 %, beispielsweise mindestens 106 %, mindestens 110 % oder mindestens 120 %, verbessert werden, verglichen mit Produkten, die nur mindestens einen Parfümstoff aber kein Cyclodextrin enthalten.

"Langfristig" bezieht sich hierbei bevorzugt auf eine Zeit nach der Anwendung des erfindungsgemäßen Produktes von mindestens einem Tag, bevorzugt mindestens 2 Tagen, bevorzugt von mindestens 3 Tagen, stärker bevorzugt von mindestens 4 Tagen, noch stärker bevorzugt von mindestens 5 Tagen, noch stärker bevorzugt von mindestens 6 Tagen, und insbesondere von mindestens 7 Tagen, beispielsweise mindestens 8 Tagen, mindestens 9 Tagen, mindestens 10 Tagen, mindestens 11 Tagen, mindestens 12 Tagen, mindestens 13 Tagen oder mindestens 14 Tagen.

Vorzugsweise ist mindestens ein Tensid in dem erfindungsgemäßen WC-Produkt enthalten. Dieses ist ausgewählt aus der Gruppe der anionischen Tenside, der nichtionischen Tenside, der amphoteren oder zwitterionischen Tenside, der kationischen Tenside sowie Mischungen derselben. Bevorzugt ist mindestens ein anionisches Tensid enthalten.

Im Rahmen der vorliegenden Erfindung stehen Fettsäuren bzw. Fettalkohole bzw. deren Derivate - soweit nicht anders angegeben - stellvertretend für verzweigte oder unverzweigte Carbonsäuren bzw. Alkohole bzw. deren Derivate mit vorzugsweise 6 bis 22 Kohlenstoffatomen, insbesondere 8 bis 20 Kohlenstoffatomen, besonders bevorzugt 10 bis 18 Kohlenstoffatomen, äußerst bevorzugt 12 bis 16 Kohlenstoffatomen, beispielsweise 12 bis 14 Kohlenstoffatomen. Erstere sind insbesondere wegen ihrer pflanzlichen Basis als auf nachwachsenden Rohstoffen basierend aus ökologischen Gründen bevorzugt, ohne jedoch die erfindungsgemäße Lehre auf sie zu beschränken. Insbesondere sind auch die beispielsweise nach der ROELENschen Oxo-Synthese erhältlichen Oxo-Alkohole bzw. deren Derivate mit vorzugsweise 7 bis 19 Kohlenstoffatomen, insbesondere 9 bis 19 Kohlenstoffatomen, besonders bevorzugt 9 bis 17 Kohlenstoffatomen, äußerst bevorzugt 11 bis 15 Kohlenstoffatomen, beispielsweise 9 bis 11, 12 bis 15 oder 13 bis 15 Kohlenstoffatomen, entsprechend einsetzbar.

Feste WC-Produkte wie WC-Steine enthalten generell vorzugsweise mindestens ein Alkylbenzolsulfonat und mindestens ein Olefinsulfonat. Daneben können weitere Tenside enthalten sein, insbesondere aus der Gruppe der anionischen und/oder nichtionischen Tenside.

Bei den Alkylbenzolsulfonaten sind dabei insbesondere solche mit etwa 12 C-Atomen im Alkylteil bevorzugt, etwa lineares Natrium-C10-13-Alkylbenzolsulfonat. Bevorzugte Olefinsulfonate weisen eine Kohlenstoffkettenlänge von 14 bis 16 auf. Das WC-Produkt enthält dabei bevorzugt 10 bis 70 Gew.-%, vorzugsweise 20 bis 65 Gew.-%, besonders bevorzugt 20 bis 30 Gew.-% Alkylbenzolsulfonat und bevorzugt 10 bis 30 Gew.-%, vorzugsweise 15 bis 30 Gew.-%, besonders bevorzugt 15 bis 25 Gew.-% Olefinsulfonat.

Als weitere anionische Tenside können im WC-Produkt aliphatische Sulfate wie Fettalkoholsulfate, Fettalkoholethersulfate, Dialkylethersulfate, Monoglyceridsulfate und aliphatische Sulfonate wie Alkansulfonate, Ethersulfonate, n-Alkylethersulfonate, Estersulfonate und Ligninsulfonate sein. Ebenfalls im Rahmen der vorliegenden Erfindung verwendbar sind Fettsäurecyanamide, Sulfosuccinate (Sulfobernsteinsäureester), insbesondere Sulfobernsteinsäuremono- und -di-C₈-C₁₈-Alkylester, Sulfosuccinamate, Sulfosuccinamide, Fettsäureisethionate, Acylaminoalkansulfonate (Fettsäuretauride), Fettsäuresarcosinate, Ethercarbonsäuren und Alkyl(ether)phosphate sowie α-Sulfofettsäuresalze, Acylglutamate, Monoglyceriddisulfate und Alkylether des Glycerindisulfats.

Bevorzugt im Rahmen der vorliegenden Erfindung sind die Fettalkoholsulfate und/oder Fettalkoholethersulfate, insbesondere die Fettalkoholsulfate. Fettalkoholsulfate sind Produkte von Sulfatierreaktionen an entsprechenden Alkoholen, während Fettalkoholethersulfate Produkte von Sulfatierreaktionen an alkoxylierten Alkoholen sind. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade. Eine weitere Ausführungsform der Alkoxylierung besteht im Einsatz von Gemischen der Alkylenoxide, bevorzugt des Gemisches von Ethylenoxid und Propylenoxid. Bevorzugte Fettalkoholethersulfate sind die Sulfate niederethoxylierter Fettalkohole mit 1 bis 4 Ethylenoxideinheiten (EO), insbesondere 1 bis 2 EO, beispielsweise 1,3 EO.

Die anionischen Tenside werden vorzugsweise als Natriumsalze eingesetzt, können aber auch als andere Alkali- oder Erdalkalimetallsalze, beispielsweise Magnesiumsalze, sowie in Form von Ammonium- oder Mono-, Di-, Tri- bzw. Tetraalkylammoniumsalzen enthalten sein, im Falle der Sulfonate auch in Form ihrer korrespondierenden Säure, z.B. Dodecylbenzolsulfonsäure.

In verschiedenen Ausführungsformen enthält die Mischung aus WC-Produkt-Inhaltsstoffen, insbesondere WC-Stein-Inhaltsstoffen, wie voranstehend definiert, mindestens eine Alkylbenzolsulfonsäure, vorzugsweise in Form ihres Natriumsalzes, in einer Menge von ungefähr 10 bis 30 Gew.-%, vorzugsweise ungefähr 20 bis 25 Gew.-%, jeweils bezogen auf das Gesamtgewicht des Produktes.

Nichtionische Tenside im Rahmen der Erfindung können Alkoxylate sein wie Polyglycolether, Fettalkoholpolyglycolether, Alkylphenolpolyglycolether, endgruppenverschlossene Polyglycolether, Mischether und Hydroxymischether und Fettsäurepolyglycolester. Ebenfalls verwendbar sind Ethylenoxid/Propylenoxid-Blockpolymere, Fettsäurealkanolamide und Fettsäurepolyglycolether. Eine weitere wichtige Klasse nichtionischer Tenside, die erfindungsgemäß verwendet werden kann, sind die Polyol-Tenside und hier besonders die Glykotenside, wie Alkylpolyglykoside und Fettsäureglucamide. Besonders bevorzugt sind die Alkylpolyglykoside, insbesondere die Alkylpolyglucoside, sowie vor allem die Fettalkoholalkoxylate (Fettalkoholpolyglycolether).

Bevorzugte Fettalkoholalkoxylate sind mit Ethylenoxid (EO) und/oder Propylenoxid (PO) alkoxylierte, unverzweigte oder verzweigte, gesättigte oder ungesättigte Cs-22-Alkohole mit einem Alkoxylierungsgrad bis zu 30, vorzugsweise ethoxylierte C₁₂₋₂₂-Fettalkohole mit einem Ethoxylierungsgrad von weniger als 30, bevorzugt 12 bis 28, insbesondere 20 bis 28, besonders bevorzugt 25, beispielsweise C₁₆₋₁₈-Fettalkoholethoxylate mit 25 EO.

Alkylpolyglykoside sind Tenside, die durch die Reaktion von Zuckern und Alkoholen nach den einschlägigen Verfahren der präparativen organischen Chemie erhalten werden können, wobei es je nach Art der Herstellung zu einem Gemisch monoalkylierter, oligomerer oder polymerer Zucker kommt. Bevorzugte Alkylpolyglykoside sind die Alkylpolyglucoside, wobei besonders bevorzugt der Alkohol ein langkettiger Fettalkohol oder ein Gemisch langkettiger Fettalkohole mit verzweigten oder unverzweigten Cs- bis Cis-Alkylketten ist und der Oligomerisierungsgrad (DP) der Zucker zwischen 1 und 10, vorzugsweise 1 bis 6, insbesondere 1,1 bis 3, äußerst bevorzugt 1,1 bis 1,7, beträgt, beispielsweise Cs -₁₀-Alkyl-1,5-glucosid (DP von 1,5).

Vorzugsweise werden Fettalkoholethoxylate in Mengen von bis zu 20 Gew.-%, besonders bevorzugt 4 bis 12 Gew.-%, besonders bevorzugt 7 bis 9 Gew.-% eingesetzt. Daneben können weitere nichtionische Tenside, etwa Fettsäuremonoalkanolamide und/oder Alkylpolyglykoside, in Mengen von bis zu 10 Gew.-% enthalten sein.

Neben den bisher genannten Tensidtypen kann die Mischung weiterhin auch Kationtenside und/oder amphotere bzw. zwitterionische Tenside enthalten.

Geeignete Amphotenside sind beispielsweise Betaine der Formel (Rⁱⁱⁱ)(R^{iv})(R^{v})N⁺CH₂COO⁻, in der Rⁱⁱⁱ einen gegebenenfalls durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀-C₁₈-Alkyl-dimethylcarboxymethylbetain und C₁₁-C₁₇-Alkylamidopropyldimethylcarboxymethylbetain.

Geeignete Kationtenside sind u.a. die quartären Ammoniumverbindungen der Formel (R^{vi})(R^{vii})(R^{viii})(R^{ix})N⁺ X⁻, in der R^{vi} bis R^{ix} für vier gleich- oder verschiedenartige, insbesondere zwei lang- und zwei kurzkettige, Alkylreste und X⁻ für ein Anion, insbesondere ein Halogenidion, stehen, beispielsweise Didecyldimethylammoniumchlorid, Alkylbenzyldidecylammoniumchlorid und deren Mischungen. Bevorzugt sind quaternäre Ammoniumverbindungen mit einer antimikrobiellen Wirkung.

In verschiedenen Ausführungsformen ist es bevorzugt, wenn, zusätzlich zu mindestens einem Parfümstoff und mindestens einem Tensid, wie jeweils voranstehend definiert, insgesamt nicht mehr als 60 Gew.-% weitere Inhaltsstoffe enthalten sind, vorzugsweise 0,01 bis 60 Gew.-%, insbesondere 0,2 bis 15 Gew.-%, jeweils bezogen auf das Gesamtgewicht des Produkts.

Zur Verstärkung der Reinigungsleistung gegenüber Kalk und Urinstein kann das WC-Produkt eine oder mehrere Säuren und/oder deren Salze enthalten. Bevorzugt werden die Säuren aus nachwachsenden Rohstoffen hergestellt. Als Säuren eignen sich daher insbesondere organische Säuren wie Ameisensäure, Essigsäure, Citronensäure, Glycolsäure, Milchsäure, Bernsteinsäure, Adipinsäure, Äpfelsäure, Weinsäure und Gluconsäure sowie Gemische derselben. Daneben können aber auch die anorganischen Säuren Salzsäure, Schwefelsäure, Phosphorsäure und Salpetersäure oder auch Amidosulfonsäure bzw. deren Mischungen eingesetzt werden. Besonders bevorzugt sind die Säuren und/oder ihre Salze ausgewählt aus der Gruppe umfassend Citronensäure, Milchsäure, Ameisensäure, ihre Salze sowie Gemische derselben. Sie werden vorzugsweise in Mengen von 0,01 bis 10 Gew.-% eingesetzt, besonders bevorzugt 0,2 bis 5 Gew.-%.

Daneben können anorganische Salze in der Mischung enthalten sein, vorzugsweise Alkali- oder Erdalkalimetallsalze, insbesondere Carbonate, Sulfate, Halogenide oder Phosphate sowie Gemische derselben. Besonders bevorzugt werden Natriumsulfat und/oder Natriumcarbonat eingesetzt. Natriumsulfat kann dabei in einer Menge von bis zu 60 Gew.-% enthalten sein, vorzugsweise 0,01 bis 60 Gew.-%, besonders bevorzugt 20 bis 60 Gew.-%, insbesondere 35 bis 55 Gew.-%. Natriumcarbonat und weitere Salze können in einer Menge von bis zu 30 Gew.-%, vorzugsweise bis zu 10 Gew.-%, besonders bevorzugt bis zu 5 Gew.-% enthalten sein.

Weiterhin können Alkalien enthalten sein. Als Basen werden vorzugsweise solche aus der Gruppe der Alkali- und Erdalkalimetallhydroxide und -carbonate, insbesondere Natriumcarbonat oder Natriumhydroxid, eingesetzt. Daneben können aber auch Ammoniak und/oder Alkanolamine mit bis zu 9 C-Atomen im Molekül verwendet werden, vorzugsweise die Ethanolamine, insbesondere Monoethanolamin.

Eine besondere Form der Reinigung stellen die Desinfektion und die Sanitation dar. In einer entsprechenden besonderen Ausführungsform der Erfindung enthält das Produkt daher einen oder mehrere antimikrobielle Wirkstoffe, vorzugsweise in einer Menge von 0,01 bis 5 Gew.-%, vorzugsweise 0,02 bis 4 Gew.-%, insbesondere 0,1 bis 3,5 Gew.-%, besonders bevorzugt 0,5 bis 3 Gew.-%, jeweils bezogen auf das Gesamtgewicht des WC-Produkts.

Die Begriffe Desinfektion, Sanitation, antimikrobielle Wirkung und antimikrobieller Wirkstoff haben im Rahmen der erfindungsgemäßen Lehre die fachübliche Bedeutung. Während Desinfektion im engeren Sinne der medizinischen Praxis die Abtötung von - theoretisch allen - Infektionskeimen bedeutet, ist unter Sanitation die möglichst weitgehende Eliminierung aller - auch der für den Menschen normalerweise unschädlichen saprophytischen - Keime zu verstehen. Hierbei ist das Ausmaß der Desinfektion bzw. Sanitation von der antimikrobiellen Wirkung des angewendeten Mittels abhängig, die mit abnehmendem Gehalt an antimikrobiellem Wirkstoff bzw. zunehmender Verdünnung des Mittels zur Anwendung abnimmt.

Erfindungsgemäß geeignet sind beispielsweise antimikrobielle Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, Iodo-2-propynyl-butyl-carbamat, Iod, Iodophore, Aktivchlor abspaltenden Verbindungen und Peroxide. Bevorzugte antimikrobielle Wirkstoffe werden vorzugsweise ausgewählt aus der Gruppe umfassend Ethanol, n-Propanol, i-Propanol, 1,3-Butandiol, Phenoxyethanol, 1,2-Propylenglykol, Glycerin, Undecylensäure, Citronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine und Natrium- Dichlorisocyanurat (DCI, 1,3-Dichlor-5H-1,3,5-triazin-2,4,6-trion Natriumsalz). Bevorzugte antimikrobiell wirkende oberflächenaktive quaternäre Verbindungen enthalten eine Ammonium-, Sulfonium-, Phosphonium-, Jodonium- oder Arsoniumgruppe. Weiterhin können auch antimikrobiell wirksame ätherische Öle eingesetzt werden, die gleichzeitig für eine Beduftung des Reinigungsmittels sorgen. Besonders bevorzugte antimikrobielle Wirkstoffe sind jedoch ausgewählt aus der Gruppe umfassend Salicylsäure, quaternäre Tenside, insbesondere Benzalkoniumchlorid, Peroxo- Verbindungen, insbesondere Natriumpercarbonat, Phthalimidoperoxyhexanoic acid oder Wasserstoffperoxid, Alkalimetallhypochlorit, Trichloroisocyanursäure, Natriumdichlorisocyanurat sowie Gemische derselben. Ganz besonders bevorzugt ist hierbei das Natriumdichlorisocyanurat.

Konservierungsstoffe können gleichfalls enthalten sein. Als solche können im Wesentlichen die bei den antimikrobiellen Wirkstoffen genannten Stoffe eingesetzt werden.

Komplexbildner (INCI Chelating Agents), auch Sequestriermittel genannt, sind Inhaltsstoffe, die Metallionen zu komplexieren und zu inaktivieren vermögen, um ihre nachteiligen Wirkungen auf die Stabilität oder das Aussehen der Produkte, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert andererseits die oxidative Zersetzung der fertigen Produkte. Zudem unterstützen die Komplexbildner die Reinigungswirkung.

Geeignet sind beispielsweise die folgenden gemäß INCI bezeichneten Komplexbildner: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Weitere geeignete WC-Produkt-Inhaltsstoffe sind Polymere. Diese können beispielsweise zur Verringerung der Kalkbildung sowie der Wiederanschmutzungsneigung (sog. Soil Repellent-Polymere) dienen. Bevorzugte Polymere sind dabei Acrylpolymere, wie sie etwa von der Firma Rhodia unter dem Handelsnamen Mirapol kommerziell erhältlich sind.

Als weitere Inhaltsstoffe können ein oder mehrere Farbstoffe (INCI Colorants) enthalten sein. Als Farbstoffe können dabei sowohl wasserlösliche als auch öllösliche Farbstoffe verwendet werden, wobei einerseits die Kompatibilität mit weiteren Inhaltsstoffen, beispielsweise Bleichmitteln, zu beachten ist und andererseits der eingesetzte Farbstoff gegenüber der WC-Keramik auch bei längerem Einwirken nicht Substantiv wirken sollte. Bevorzugt kann ein wasserlöslicher Farbstoff sein, der das Spülwasser färbt, beispielsweise eine blaue Farbe. Das mit diesem Farbstoff gefärbte Spülwasser bleibt durch das Nachlaufen nach Beendigung des eigentlichen Spülvorgangs in ausreichender Konzentration im Toilettensumpf, also in dem im Toilettenbecken stehenbleibenden Rest Spülwasser stehen, um diesem eine, beispielsweise blaue, Farbe zu verleihen. Die Farbstoffe sind vorzugsweise in einer Menge von 0,0001 bis 0,1 Gew.-%, insbesondere 0,0005 bis 0,05 Gew.-%, besonders bevorzugt 0,001 bis 0,01 Gew.-%, enthalten.

Weiterhin können weitere Wirkstoffe zur Verhinderung oder Verringerung von Schlechtgerüchen, sog. Malodor Repellents, eingesetzt werden, wobei die weiteren Wirkstoffe zur Verhinderung oder Verringerung von Schlechtgerüchen keine Cyclodextrine sind. Bei diesen weiteren Wirkstoffen handelt es sich in der Regel um Stoffe, die die flüchtigen, den Schlechtgeruch erzeugenden Stoffe adsorbieren, komplexieren, oxidieren oder mit ihnen Einschlussverbindungen bilden, so dass sie geruchlich inaktiviert werden (sog. Desodorantien).

In den erfindungsgemäßen Produkten können ggf. wasserlösliche und/oder wasserunlösliche Builder enthalten sein. Dabei sind wasserlösliche Builder bevorzugt, da sie in der Regel weniger dazu tendieren, auf harten Oberflächen unlösliche Rückstände zu hinterlassen. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen.

Erfindungsgemäß können weiterhin Bleichmittel eingesetzt werden. Geeignete Bleichmittel umfassen Peroxo-Verbindungen, insbesondere Peroxide, Persäuren, Percarbonate und/oder Perborate, besonders bevorzugt sind Natriumpercarbonat, Phthalimidoperoxyhexansäure oder Wasserstoffperoxid. Alkalimetallhypochlorite wie das Natriumhypochlorit sind dagegen bei sauer formulierten Reinigungsmitteln aufgrund der Freisetzung giftiger Chlorgas-Dämpfe weniger geeignet, kann jedoch in alkalisch eingestellten Reinigungsmitteln eingesetzt werden. Ebenfalls geeignet sind Trichloroisocyanursäure und insbesondere Natriumdichlorisocyanurat. Unter Umständen kann neben dem Bleichmittel auch ein Bleichaktivator vonnöten sein.

Geeignete Korrosionsinhibitoren (INCI Corrosion Inhibitors) sind beispielsweise folgende gemäß INCI benannte Substanzen: Cyclohexylamine, Diammonium Phosphate, Dilithium Oxalate, Dimethylamino Methylpropanol, Dipotassium Oxalate, Dipotassium Phosphate, Disodium Phosphate, Disodium Pyrophosphate, Disodium Tetrapropenyl Succinate, Hexoxyethyl Diethylammonium, Phosphate, Nitromethane, Potassium Silicate, Sodium Aluminate, Sodium Hexametaphosphate, Sodium Metasilicate, Sodium Molybdate, Sodium Nitrite, Sodium Oxalate, Sodium Silicate, Stearamidopropyl Dimethicone, Tetrapotassium Pyrophosphate, Tetrasodium Pyrophosphate, Triisopropanolamine.

Die als Abspülregulatoren bezeichneten Substanzen dienen in erster Linie dazu, den Verbrauch der Produkte während des Einsatzes so zu steuern, dass die vorgesehene Standzeit eingehalten wird. Als Regulatoren eignen sich vorzugsweise feste langkettige Fettsäuren, wie Stearinsäure, aber auch Salze solcher Fettsäuren, Fettsäureethanolamide, wie Kokosfettsäuremonoethanolamid, oder feste Polyethylenglykole, wie solche mit Molekulargewichten zwischen 10000 und 50000.

Zur Verbesserung der Verarbeitbarkeit bei der erfindungsgemäßen Herstellung kann dem Produkt ein Wirkstoff zur Verringerung der Klebrigkeit zugesetzt werden. So verbessert die Zugabe von Dolomitpulver oder Titandioxidpulver mit feiner Partikelgrößenverteilung das Verarbeitungsverhalten und reduziert deutlich Abrieb bzw. Klebrigkeit. Die Ergebnisse mit solchen Wirkstoffen sind besser als mit anderen üblichen Maßnahmen, beispielsweise Beschichten der Produkte mit einem Gleitmittel, Abpudern oder Beschichten der Formwalzen mit Teflon.

Weitere geeignete Bestandteile für WC-Produkte wie WC-Steine sind Enzyme, vorzugsweise Proteasen, Lipasen, Amylasen, Hydrolasen und/oder Cellulasen. Sie können in jeder nach dem Stand der Technik etablierten Form zugesetzt werden. Hierzu gehören Lösungen der Enzyme, vorteilhafterweise möglichst konzentriert, wasserarm und/oder mit Stabilisatoren versetzt. Alternativ können die Enzyme verkapselt werden, beispielsweise durch Sprühtrocknung oder Extrusion der Enzymlösung zusammen mit einem, vorzugsweise natürlichen, Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Vorteilhafterweise sind derartige Granulate, beispielsweise durch Aufbringen polymerer Filmbildner, staubarm und aufgrund der Beschichtung lagerstabil.

Weiterhin können in enzymhaltigen Produkten Enzymstabilisatoren vorhanden sein, um ein in einem erfindungsgemäßen Produkt enthaltenes Enzym vor Schädigungen wie beispielsweise Inaktivierung, Denaturierung oder Zerfall etwa durch physikalische Einflüsse, Oxidation oder proteolytische Spaltung zu schützen. Als Enzymstabilisatoren sind, jeweils in Abhängigkeit vom verwendeten Enzym, insbesondere geeignet: Benzamidin-Hydrochlorid, Borax, Borsäuren, Boronsäuren oder deren Salze oder Ester, vor allem Derivate mit aromatischen Gruppen, etwa substituierte Phenylboronsäuren beziehungsweise deren Salze oder Ester; Peptidaldehyde (Oligopeptide mit reduziertem C-Terminus), Aminoalkohole wie Mono-, Di-, Triethanol- und -Propanolamin und deren Mischungen, aliphatische Carbonsäuren bis zu C12, wie Bernsteinsäure, andere Dicarbonsäuren oder Salze der genannten Säuren; endgruppenverschlossene Fettsäureamidalkoxylate; niedere aliphatische Alkohole und vor allem Polyole, beispielsweise Glycerin, Ethylenglykol, Propylenglykol oder Sorbit; sowie Reduktionsmittel und Antioxidantien wie Natrium-Sulfit und reduzierende Zucker. Weitere geeignete Stabilisatoren sind aus dem Stand der Technik bekannt. Bevorzugt werden Kombinationen von Stabilisatoren verwendet, beispielsweise die Kombination aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen.

In verschiedenen Ausführungsformen können WC-Steine mehrschichtig sein und beispielsweise aus unterschiedlich zusammengesetzten Massen hergestellt werden, wobei eine der Massen von der oder den anderen Massen ganz oder teilweise umschlossen ist. So kann beispielsweise die innere Masse eine höhere Parfümkonzentration aufweisen als die äußere, um während der Gebrauchsdauer einen gleichbleibenden Dufteindruck bei abnehmendem Außenumfang zu gewährleisten, oder aber die innere Masse enthält einen anderen Duftstoff als die äußere.

Daneben können auch andere Wirkstoffe in unterschiedliche Schichten eingearbeitet werden, die je nach Abspülgrad zu unterschiedlichen Zeiten freigesetzt werden. Ein solch schichtweiser Aufbau ist prinzipiell auch bei den hierin beschriebenen WC-Produkten, insbesondere WC-Steinen möglich.

Ein erfindungsgemäßes WC-Produkt, insbesondere ein WC-Stein, kann beispielsweise durch ein im Folgenden beschriebenes Verfahren hergestellt werden, ohne darauf beschränkt zu sein:
(a) die Bereitstellung mindestens einer Mischung aus WC-Produkt-Inhaltsstoffen;
(b) der Extrusion der mindestens einen Mischung; und
(c) der anschließenden Verformung, um einen geformten Körper zu erhalten, beispielsweise einen kugelförmigen Körper. In einem ähnlichen Verfahren können auch mehrschichtige WC-Produkte, insbesondere mehrschichtige WC-Steine hergestellt werden.

Die Formbarkeit der Masse lässt sich durch die Zugabe einer geringen Flüssigkeitsmenge einstellen. Als Flüssigkeiten können insbesondere Wasser, Dipropylenglykol oder Paraffin in einer Menge von 0,1 bis 1 Gew.-% eingesetzt werden.

Ein weiterer Aspekt der Erfindung ist die Verwendung eines erfindungsgemäßen WC-Produktes wie hierin beschrieben zur Reduzierung von Schlechtgerüchen und/oder zur Beduftung, insbesondere in der Toilette und optional in dem umgebenden Raum.

In einem weiteren Aspekt betrifft die Erfindung ein Verfahren zur Reduzierung von Schlechtgerüchen und/oder zur Beduftung, insbesondere in der Toilette und optional in dem umgebenden Raum, wobei in mindestens einem Verfahrensschritt ein erfindungsgemäßes WC-Produkt wie hierin beschrieben angewendet wird.

Ein letzter Aspekt der Erfindung ist die Verwendung von mindestens einem Cyclodextrin zur Reduzierung von Schlechtgerüchen und/oder zur Verbesserung des Dufteindrucks eines Produktes, in dem das Cyclodextrin enthalten ist, wobei das Cyclodextrin in einem WC-Produkt enthalten ist, bevorzugt einem WC-Reiniger oder WC-Erfrischer, insbesondere einem WC-Stein oder Duftspüler, und bevorzugt in der Toilette angewendet wird.

Es ist bevorzugt, dass die Verwendung des erfindungsgemäßen WC-Produktes oder des mindestens einen Cyclodextrins in einem WC-Produkt, insbesondere einem (festen) WC-Stein, einen vorliegenden Schlechtgeruch um mindestens 5 % reduziert, stärker bevorzugt um mindestens 10 %, noch stärker bevorzugt um mindestens 15 %, noch stärker bevorzugt um mindestens 20 %, noch stärker bevorzugt um mindestens 25 %, noch stärker bevorzugt um mindestens 30 %, noch stärker bevorzugt um mindestens 35 %, noch stärker bevorzugt um mindestens 40 %, noch stärker bevorzugt um mindestens 45 %, noch stärker bevorzugt um mindestens 50 %, noch stärker bevorzugt um mindestens 55 %, noch stärker bevorzugt um mindestens 60 %, noch stärker bevorzugt um mindestens 65 %, noch stärker bevorzugt um mindestens 70 %, noch stärker bevorzugt um mindestens 75 %, noch stärker bevorzugt um mindestens 80 %, noch stärker bevorzugt um mindestens 85 %, noch stärker bevorzugt um mindestens 90 %, noch stärker bevorzugt um mindestens 91 %, noch stärker bevorzugt um mindestens 92 %, noch stärker bevorzugt um mindestens 93 %, noch stärker bevorzugt um mindestens 94 %, noch stärker bevorzugt um mindestens 95 %, noch stärker bevorzugt um mindestens 96 %, noch stärker bevorzugt um mindestens 97 %, noch stärker bevorzugt um mindestens 98 %, noch stärker bevorzugt um mindestens 99 % und insbesondere um 100 %, wobei mit 100 % die vollständige Beseitigung des Schlechtgeruchs gemeint ist.

In besonders bevorzugte Ausführungsformen wird der Schlechtgeruch um mindestens 25 % reduziert, bevorzugt um mindestens 26 %, stärker bevorzugt um mindestens 30 %, stärker bevorzugt um mindestens 33 %, noch stärker bevorzugt um mindestens 40 %, noch stärker bevorzugt um mindestens 42 %, noch stärker bevorzugt um mindestens 50 %, noch stärker bevorzugt um mindestens 52 %, noch stärker bevorzugt um mindestens 55 %, noch stärker bevorzugt um mindestens 56 %, noch stärker bevorzugt um mindestens 60 % und insbesondere um mindestens 63 %.

In verschiedenen Ausführungsformen der beschriebenen Verwendungen und Verfahren geht die Reduzierung des Schlechtgeruches beispielsweise so weit, dass der Schlechtgeruch (nahezu) vollständig beseitigt wird, bevorzugt bedeutet das, dass der Schlechtgeruch zu mindestens 95 % beseitigt wird, stärker bevorzugt zu mindestens 96 %, noch stärker bevorzugt zu mindestens 97 %, noch stärker bevorzugt zu mindestens 98 %, noch stärker bevorzugt zu mindestens 99% und insbesondere zu 100 %.

Die Verbesserung des Dufteindruckes des Produktes kann sich beispielsweise sowohl auf den Duft des Produktes vor der Anwendung sowie auf den Dufteindruck, der während der Anwendung des Produktes freigesetzt wird, beispielsweise beim Abspülen der Toilette, wenn das Wasser mit einem WC-Produkt, das in der Toilette befestigt ist, in Kontakt kommt, beziehen. Bevorzugt hält sich der Dufteindruck über eine möglichst lange Zeit, wie weiter oben beschrieben.

Bevorzugt ist das mindestens eine Cyclodextrin ein α-Cydodextrin, ein β-Cyclodextrin, ein γ-Cyclodextrin, ein alkyliertes α-Cydodextrin, ein alkyliertes β-Cyclodextrin, ein alkyliertes γ-Cyclodextrin, ein hydroxyalkyliertes α-Cydodextrin, ein hydroxyalkyliertes β-Cyclodextrin oder ein hydroxyalkyliertes γ-Cyclodextrin, oder eine Mischung davon ist, stärker bevorzugt β-Cyclodextrin, alkyliertes β-Cyclodextrin, hydroxyalkyliertes β-Cyclodextrin oder eine Mischung davon, insbesondere methyliertes β-Cyclodextrin oder hydroxyalkyliertes β-Cyclodextrin oder eine Mischung davon, beispielsweise Methyl-β-Cyclodextrin oder Hydroxypropyl-β-Cyclodextrin oder eine Mischung davon.

Das mindestens eine Cyclodextrin wird bevorzugt in einer Menge von 0,01 bis 30 Gew.-%, bevorzugt 0,01 bis 25 Gew.-%, stärker bevorzugt 0,01 bis 20 Gew.-%, noch stärker bevorzugt 0,01 bis 15 Gew.-%, noch stärker bevorzugt 0,01 bis 10 Gew.-%, insbesondere 0,01 bis 5 Gew.-%, beispielsweise 0,01 bis 2 Gew.-% oder 0,01 bis 1 Gew.-% wie z.B. 0,05 Gew.-%, bezogen auf das Gesamtgewicht des WC-Produktes, in dem WC-Produkt eingesetzt.

Es versteht sich, dass alle Ausführungsformen, die hier in Bezug auf die WC-Produkte offenbart werden, in gleicher oder ähnlicherWeise auf die Verfahren und Verwendungen dieser Produkte anwendbar sind, und umgekehrt.

Die folgenden Beispiele dienen zur Veranschaulichung der vorliegenden Erfindung. Da diese Beispiele nur zur Veranschaulichung dienen, sollte die Erfindung nicht als darauf beschränkt angesehen werden.

### Beispiele

Der Test für WC Produkte, insbesondere WC-Steine, wird in einer Plexiglaskammer mit einem Volumen von 27 L durchgeführt. Zur Durchführung wird eine Haltevorrichtung für den Klostein sowie eine Schale zur Nachstellung des Toilettensumpfes sowie ein Filterpapier als Schlechtgeruchträger benötigt.

Der Schlechtgeruch, eine Mischung aus Dimethylsulfid, Indol, Skatol, Buttersäure, Isovaleriansäure, und Valeriansäure, wird auf das Filterpapier pipettiert und dient als Fäkalienersatz.

Eine zuvor kalkulierte Menge an Klostein (Abspülmenge einer Spülung) wird in einem Behälter aufgelöst und in die Schale dosiert wo zuvor 280 mL Leitungswasser vorgelegt wurden. Der Träger mit dem Klostein wird nun mit Wasser angesprüht um die Abspülung zu simulieren und den Duft freizusetzen. Danach wird die Testkammer für 5 Minuten dicht verschlossen.

Im Anschluss wird die Plexiglaskammer geöffnet und der Gasraum durch jeweils 10 geschulte Personen auf einer Skala von 1 = sehr schwache Intensität bis 10 = sehr starke Intensität geruchlich bewertet. Es ergaben sich die in der folgenden Tabelle angegeben Ergebnisse als Mittelwerte.

**Tabelle 1: Geruchsintensität von Parfüm und Schlechtgeruch nach 5 Minuten.**

| | Parfüm-intensität | Schlechtgeruchintensität |
|---|---|---|
| SVR Grundmasse ohne Parfüm | 0 | 8,8 |
| SVR Grundmasse + Parfüm | 6,5 | 5,7 |
| SVR Grundmasse + Parfüm + 0,05% CavasolW7M | 7,1 | 3,8 |
| SVR Grundmasse + Parfüm + 0,05% CavasolW7HP | 6,8 | 4,2 |

**Tabelle 2: Geruchsintensität von Parfüm und Schlechtgeruch nach sieben Tagen.**

| | Parfüm-intensität | Schlechtgeruchintensität |
|---|---|---|
| SVR Grundmasse ohne Parfüm | 0 | 6,5 |
| SVR Grundmasse + Parfüm | 2,0 | 5,0 |
| SVR Grundmasse + Parfüm + 0,05% CavasolW7M | 4,1 | 2,4 |
| SVR Grundmasse + Parfüm + 0,05% CavasolW7HP | 3,5 | 2,9 |

## Patentansprüche

1. Ein WC-Produkt, bevorzugt zur Reduzierung von Schlechtgerüchen und/oder zur Beduftung, wobei das WC-Produkt mindestens ein Cyclodextrin umfasst.

2. Das WC-Produkt gemäß Anspruch 1, wobei das WC-Produkt das mindestens eine Cyclodextrin in einer Menge von 0,01 bis 30 Gew.-% enthält, bevorzugt 0,01 bis 25 Gew.-%, stärker bevorzugt 0,01 bis 20 Gew.-%, noch stärker bevorzugt 0,01 bis 15 Gew.-%, noch stärker bevorzugt 0,01 bis 10 Gew.-%, insbesondere 0,01 bis 5 Gew.-%, bezogen auf das Gesamtgewicht des WC-Produktes.

3. Das WC-Produkt gemäß Anspruch 1 oder 2, wobei das mindestens eine Cyclodextrin ein α-Cyclodextrin, ein β-Cyclodextrin, ein γ-Cyclodextrin, ein alkyliertes α-Cydodextrin, ein alkyliertes β-Cyclodextrin, ein alkyliertes γ-Cyclodextrin, ein hydroxyalkyliertes α-Cydodextrin, ein hydroxyalkyliertes β-Cyclodextrin oder ein hydroxyalkyliertes γ-Cyclodextrin, oder eine Mischung davon ist.

4. Das WC-Produkt gemäß einem der Ansprüche 1 bis 3, wobei das Cyclodextrin β-Cyclodextrin, alkyliertes β-Cyclodextrin, hydroxyalkyliertes β-Cyclodextrin oder eine Mischung davon ist.

5. Das WC-Produkt gemäß einem der Ansprüche 1 bis 4, wobei das Cyclodextrin bevorzugt methyliertes β-Cyclodextrin oder hydroxyalkyliertes β-Cyclodextrin oder eine Mischung davon ist, bevorzugt Methyl-β-Cyclodextrin oder Hydroxypropyl-β-Cyclodextrin oder eine Mischung davon.

6. Das WC-Produkt gemäß einem der Ansprüche 1 bis 5, wobei das WC-Produkt ein WC-Reiniger oder ein WC-Erfrischer ist, bevorzugt ein WC-Stein oder ein Duftspüler, insbesondere ein WC-Stein.

7. Das WC-Produkt gemäß einem der Ansprüche 1 bis 6, wobei das WC-Produkt fest ist, insbesondere ein fester WC-Stein.

8. Das WC-Produkt gemäß einem der Ansprüche 1 bis 7, wobei das WC-Produkt einen Schlechtgeruch um mindestens 25 % reduziert, bevorzugt um mindestens 30 %, stärker bevorzugt um mindestens 33 %, noch stärker bevorzugt um mindestens 40 %, noch stärker bevorzugt um mindestens 42 %, noch stärker bevorzugt um mindestens 50 %, noch stärker bevorzugt um mindestens 52 %, noch stärker bevorzugt um mindestens 55 %, noch stärker bevorzugt um mindestens 56 % und insbesondere um mindestens 60 %, beispielsweise um mindestens 63 %.

9. Das WC-Produkt gemäß einem der Ansprüche 1 bis 8, wobei das WC-Produkt mindestens einen weiteren Bestandteil enthält, bevorzugt mindestens zwei weitere Bestandteile, ausgewählt aus der Gruppe bestehend aus Tensiden, Farbstoffen, Parfümstoffen, Abspülregulatoren, Feuchthaltemitteln, Bleichmitteln, Buildern, Säuren, Basen, Lösungsmitteln, antimikrobiellen Wirkstoffen, Polymeren, Salzen, Verdickungsmitteln, Konservierungsstoffen, Komplexbildnern, weiteren Wirkstoffen zur Verringerung von Schlechtgerüchen, Parfümboostern, Füllstoffen, Bleichmitteln, Korrosionsinhibitoren, Enzymen, Mikroorganismen, Wirkstoffen zur Biofilmentfernung, Wirkstoffen zur Inhibierung der Kalkablagerung, Wirkstoffen zur Verminderung der Schmutzhaftung, Wirkstoffen zur Verbesserung der Verarbeitbarkeit, Wirkstoffen zur Verringerung der Klebrigkeit und Mischungen davon.

10. Das WC-Produkt gemäß einem der Ansprüche 1 bis 9, wobei das WC-Produkt mindestens einen Parfümstoff oder eine Mischung mehrerer Parfümstoffe umfasst, bevorzugt in einer Menge von 0,01 bis 10 Gew.-%, insbesondere 0,05 bis 8 Gew.-%, insbesondere 0,1 bis 5 Gew.-%.

11. Verwendung des WC-Produktes gemäß einem der Ansprüche 1 bis 10 zur Reduzierung von Schlechtgerüchen und/oder zur Beduftung, insbesondere in der Toilette und optional in dem umgebenden Raum.

12. Ein Verfahren zur Reduzierung von Schlechtgerüchen und/oder zur Beduftung, insbesondere in der Toilette und optional in dem umgebenden Raum, wobei in mindestens einem Verfahrensschritt ein WC-Produkt gemäß einem der Ansprüche 1 bis 10 angewendet wird.

13. Verwendung von mindestens einem Cyclodextrin zur Reduzierung von Schlechtgerüchen und/oder zur Verbesserung des Dufteindrucks eines Produktes, in dem das Cyclodextrin enthalten ist, wobei das Cyclodextrin in einem WC-Produkt enthalten ist, bevorzugt einem WC-Reiniger oder WC-Erfrischer, insbesondere einem WC-Stein, und bevorzugt in der Toilette angewendet wird.

14. Die Verwendung gemäß Anspruch 13, wobei das mindestens eine Cyclodextrin ein α-Cyclodextrin, ein β-Cyclodextrin, ein γ-Cyclodextrin, ein alkyliertes α-Cyclodextrin, ein alkyliertes β-Cyclodextrin, ein alkyliertes γ-Cyclodextrin, ein hydroxyalkyliertes α-Cyclodextrin, ein hydroxyalkyliertes β-Cyclodextrin oder ein hydroxyalkyliertes γ-Cyclodextrin, oder eine Mischung davon ist, bevorzugt β-Cyclodextrin, alkyliertes β-Cyclodextrin, hydroxyalkyliertes β-Cyclodextrin oder eine Mischung davon, stärker bevorzugt methyliertes β-Cyclodextrin oder hydroxyalkyliertes β-Cyclodextrin oder eine Mischung davon, insbesondere Methyl-β-Cyclodextrin oder Hydroxypropyl-β-Cyclodextrin oder eine Mischung davon.

15. Die Verwendung gemäß Anspruch 13 oder 14, wobei das mindestens eine Cyclodextrin in einer Menge von 0,01 bis 30 Gew.-% bevorzugt 0,01 bis 25 Gew.-%, stärker bevorzugt 0,01 bis 20 Gew.-%, noch stärker bevorzugt 0,01 bis 15 Gew.-%, noch stärker bevorzugt 0,01 bis 10 Gew.-%, insbesondere 0,01 bis 5 Gew.-%, in dem WC-Produkt enthalten ist, bezogen auf das Gesamtgewicht des WC-Produktes.
